# EUROPEAN PATENT APPLICATION

(11) **EP 4 349 357 A1**
(43) Date of publication of application: **10.04.2024**
(21) Application number: 22811297.5
(22) Date of filing: 24.05.2022
(51) Int. Cl.: A61K 36/22, A23K 10/37, A23K 50/10, A23K 50/30, A23K 50/75, A61K 31/05, A61K 31/122, A61K 31/60, A61P 1/00, A61P 29/00, A61P 43/00

(54) **ANTI-INFLAMMATORY AGENT FOR INTESTINAL CELLS OF DOMESTIC ANIMAL**

(30) Priority: 28.05.2021 JP 2021090143
(71) Applicant: SDS BIOTECH K.K., Tokyo 101-0022 (JP)
(72) Inventor: HIYAMUTA, Shuichi, Tokyo 100-8321 (JP); OGAWA, Ryo, Tokyo 100-8321 (JP); INDO, Yuki, Tokyo 100-8321 (JP); KITAZAWA, Haruki, Sendai-shi, Miyagi 980-8577 (JP)
(74) Representative: Vossius & Partner Patentanwälte Rechtsanwälte mbB
(86) International application number: PCT/JP2022/021171
(87) International publication number: WO 2022/250027

(57) **Abstract**

An agent for suppressing inflammation in the intestinal cell of a non-human animal comprises cashew nut shell liquid, heated cashew nut shell liquid, anacardic acid, cardanol, and/or cardol, and vitamin K.

## Description

### TECHNICAL FIELD

The present invention relates to: an agent and feed for suppressing inflammation of the intestinal cells of a ruminant such as cattle, or a nonhuman animal such as a pig or poultry; and a method for suppressing inflammation of the intestinal cells of the nonhuman animal using the agent and the feed.

### BACKGROUND ART

The health condition maintenance and disease control of domestic animals are important factors for improving the productivity of the domestic animals such as ruminants, pigs, and poultry.

LPS (lipopolysaccharide) and the like which are the decomposition products of pathogenic bacteria cause intestinal inflammation in the intestines of the domestic animals, resulting in various gastrointestinal diseases including diarrhea and colitis, decreases in resistance and immunity due to nutritional deprivation, and susceptibility to diseases, caused by the decreases, which have been greatly problematic in the field of animal industry.

In contrast, live microbial agents such as *Bacillus* bacteria and bifidobacteria have also been used in the animal industry for the purpose of intestinal regulation effects. However, there have not been any natural product formulations confirmed to have the effects of suppressing the secretory of inflammatory cytokines.

Known effects of cashew nut shell liquid on domestic animals include the effect of improving rumen fermentation (Patent Document 1), the effect of preventing tympanites (Patent Document 2), the effect of preventing coccidiosis (Patent Document 3), and the effect of preventing acidosis (Patent Document 4).

Patent Document 3 discloses an agent for preventing coccidiosis, including at least one selected from vitamin A, vitamin B, vitamin C, vitamin D, and vitamin E, and cashew nut shell liquid and/or anacardic acids. However, Patent Document 3 does not disclose the combination with vitamin K. None of the above-described documents discloses suppression of the intestinal inflammation of domestic animals.

### PRIOR ART DOCUMENTS

### Patent Documents

Patent Document 1: WO 2008/149992
Patent Document 2: WO 2008/149994
Patent Document 3: WO 2009/139468
Patent Document 4: WO 2010/053085

### SUMMARY OF THE INVENTION

An object of the present invention is to provide a material for suppressing the intestinal inflammation of a non-human animal.

The inventors intensively studied to solve the above-described problems and consequently found that an action of effectively suppressing inflammation of the intestinal cells of an animal is provided by combining cashew nut shell liquid (hereinafter, may be abbreviated as "CNSL") with vitamin K. The inventors completed the present invention on the basis of such findings.

That is, the present invention is as follows.
[1] A method of suppressing inflammation in an intestinal cells of a nonhuman animal, comprising administering cashew nut shell liquid, heated cashew nut shell liquid, anacardic acid, cardanol, and/or cardol, and vitamin K to a nonhuman animal.
[2] The method according to [1], comprising: mixing a feed with cashew nut shell liquid, heated cashew nut shell liquid, anacardic acid, cardanol, and/or cardol, and vitamin K; and administering the feed to the non-human animal.
[3] The method according to [2], comprising: mixing the feed with 1 ppm to 1000 ppm (weight) of cashew nut shell liquid, heated cashew nut shell liquid, anacardic acid, cardanol, and/or cardol, and 1 to 20 ppm (weight) of vitamin K; and administering the feed to the nonhuman animal.
[4] The method of suppressing inflammation in intestinal cells according to any of [1] to [3], wherein the nonhuman animal is a mammal.
[5] The method of suppressing inflammation in intestinal cells according to any of [1] to [3], wherein the non-human animal is poultry, a pig, or a ruminant.
[6] The method of suppressing inflammation in intestinal cells according to any of [1] to [3], wherein the non-human animal is a cattle.
[7] The method of suppressing inflammation in intestinal cells in a non-human animal according to any of [1] to [6], wherein the administration of cashew nut shell liquid, heated cashew nut shell liquid, anacardic acid, cardanol, and/or cardol, and vitamin K results in suppression of production of an inflammatory cytokine in intestinal cells to suppress the inflammation in the intestinal cells of the non-human animal.
[8] The method according to [7], wherein the inflammatory cytokine is one or more of TNFα, IL-6, IL-8, and MCP-1.
[9] The method according to any of [1] to [8], wherein the vitamin K is vitamin K1.
[10] The method according to any of [1] to [9], said method comprising administering cashew nut shell liquid and/or anacardic acid, and vitamin K to a nonhuman animal.
[11] An agent for suppressing intestinal inflammation of a non-human animal, comprising, as active components, cashew nut shell liquid, heated cashew nut shell liquid, anacardic acid, cardanol, and/or cardol, and vitamin K.
[12] The agent for suppressing intestinal inflammation according to [11], wherein the vitamin K is vitamin K1.
[13] The agent for suppressing intestinal inflammation according to [11] or [12h], wherein said agent comprises, as active components, cashew nut shell liquid and/or anacardic acid, and vitamin K.
[14] An agent for suppressing production of an inflammatory cytokine in intestinal cells of a nonhuman animal, comprising, as active components, cashew nut shell liquid, heated cashew nut shell liquid, anacardic acid, cardanol, and/or cardol, and vitamin K.
[15] The agent for suppressing production of an inflammatory cytokine according to [14], wherein the vitamin K is vitamin K1.
[16] The agent for suppressing production of an inflammatory cytokine according to [14] or [15], wherein said agent comprises, as active components, cashew nut shell liquid and/or anacardic acid, and vitamin K.
[17] A feed for suppressing intestinal inflammation of a non-human animal, comprising cashew nut shell liquid, heated cashew nut shell liquid, anacardic acid, cardanol, and/or cardol, and vitamin K.
[18] The feed according to [17], comprising 1 ppm to 1000 ppm (weight) of cashew nut shell liquid, heated cashew nut shell liquid, anacardic acid, cardanol, and/or cardol, and 1 to 20 ppm (weight) of vitamin K.
[19] The feed according to [17], comprising 5 ppm to 100 ppm (weight) of cashew nut shell liquid, heated cashew nut shell liquid, anacardic acid, cardanol, and/or cardol, and 1 to 10 ppm (weight) of vitamin K.
[20] The feed according to [17], wherein the weight ratio of the content of cashew nut shell liquid, heated cashew nut shell liquid, anacardic acid, cardanol, and/or the cardol and the content of vitamin K is in the range of 30:1 to 30:10.
[21] The feed according to any of [17] to [20], said feed comprising cashew nut shell liquid and/or anacardic acid, and vitamin K.

### EFFECTS OF THE INVENTION

In accordance with the present invention, production of an inflammatory cytokine secreted from intestinal cells can be suppressed by administering an agent or feed comprising cashew nut shell liquid, heated cashew nut shell liquid, anacardic acid, cardanol, and/or cardol, and vitamin K to a non-human animal such as a ruminant, a pig, or poultry. As a result, the onset and symptomatic worsening of inflammation in the intestinal cells can be suppressed. As a result, the health condition of the non-human animal such as a domestic animal can be maintained to improve productivity. The cashew nut shell liquid has an advantage of acting in a low dose and also having high safety.

### BRIEF DESCRIPTION OF DRAWINGS

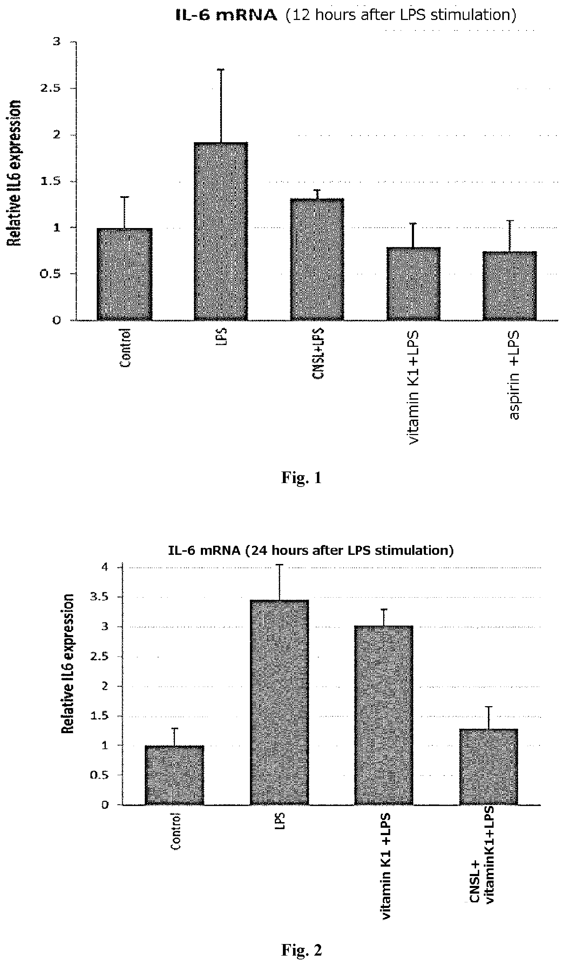

[Figure 1] Figure 1 is a graph indicating the results (RT-PCR) of the analysis of the expression of an inflammatory cytokine gene (IL-6) in the case of adding various substances to bovine intestinal epithelial cells (after 12 hours). Their effects on LPS-stimulated increase in the expression of the inflammatory cytokine gene were examined. The ordinate indicates a relative expression level on the assumption that an expression level without addition of the various substances and without the LPS stimulation is set at 1.
[Figure 2] Figure 2 is a graph indicating the results (RT-PCR) of the analysis of the expression of an inflammatory cytokine gene (IL-6) in the case of adding various substances to bovine intestinal epithelial cells (after 24 hours). Their effects on LPS-stimulated increase in the expression of the inflammatory cytokine gene were examined. The ordinate indicates a relative expression level on the assumption that an expression level without addition of the various substances and without the LPS stimulation is set at 1.

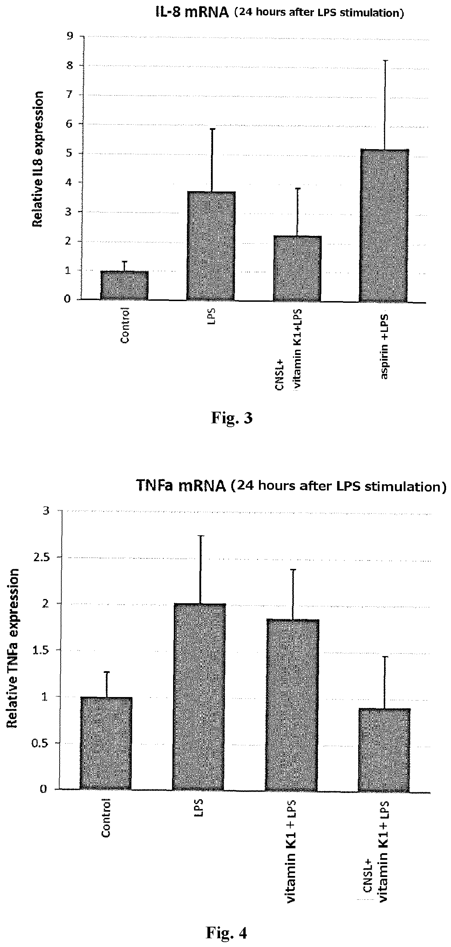

[Figure 3] Figure 3 is a graph indicating the results (RT-PCR) of the analysis of the expression of an inflammatory cytokine gene (IL-8) in the case of adding various substances to bovine intestinal epithelial cells (after 24 hours). Their effects on LPS-stimulated increase in the expression of the inflammatory cytokine gene were examined. The ordinate indicates a relative expression level on the assumption that an expression level without addition of the various substances and without the LPS stimulation is set at 1.
[Figure 4] Figure 4 is a graph indicating the results (RT-PCR) of the analysis of the expression of an inflammatory cytokine gene (TNF-α) in the case of adding various substances to bovine intestinal epithelial cells (after 24 hours). Their effects on LPS-stimulated increase in the expression of the inflammatory cytokine gene were examined. The ordinate indicates a relative expression level on the assumption that an expression level without addition of the various substances and without the LPS stimulation is set at 1.

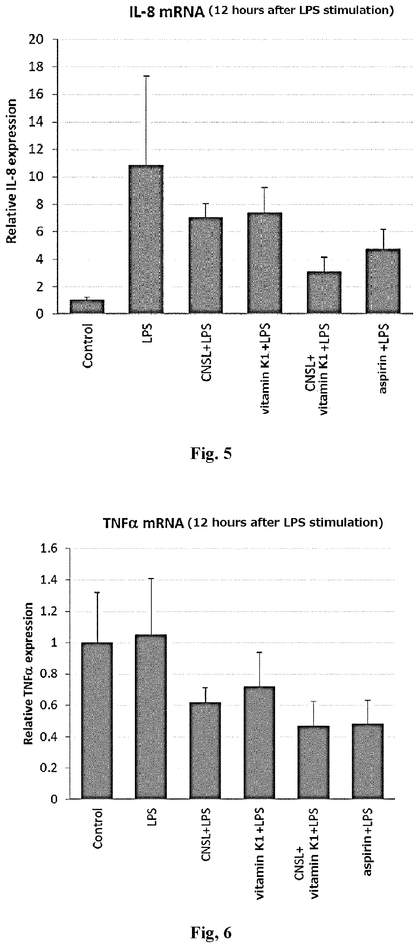

[Figure 5] Figure 5 is a graph indicating the results (RT-PCR) of the analysis of the expression of an inflammatory cytokine gene (IL-8) in the case of adding various substances to bovine intestinal epithelial cells (after 12 hours). Their effects on LPS-stimulated increase in the expression of the inflammatory cytokine gene were examined. The ordinate indicates a relative expression level on the assumption that an expression level without addition of the various substances and without the LPS stimulation is set at 1.
[Figure 6] Figure 6 is a graph indicating the results (RT-PCR) of the analysis of the expression of an inflammatory cytokine gene (TNF-α) in the case of adding various substances to bovine intestinal epithelial cells (after 12 hours). Their effects on LPS-stimulated increase in the expression of the inflammatory cytokine gene were examined. The ordinate indicates a relative expression level on the assumption that an expression level without addition of the various substances and without the LPS stimulation is set at 1.

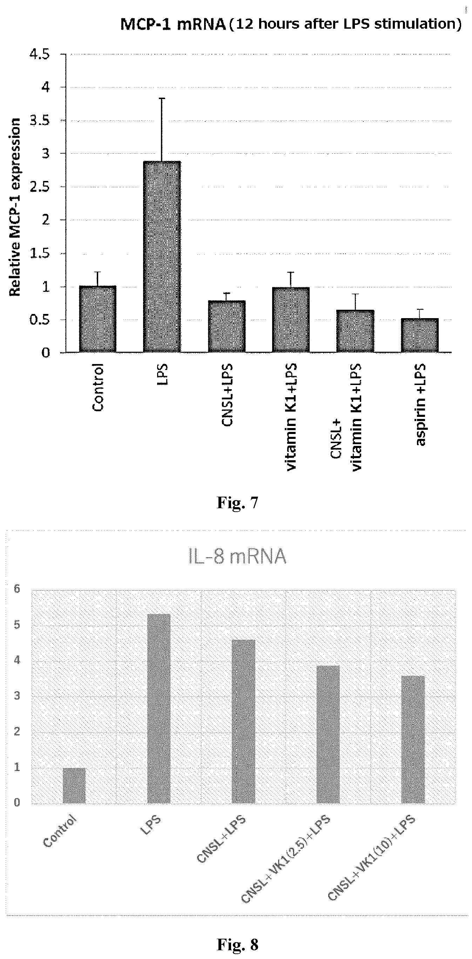

[Figure 7] Figure 7 is a graph indicating the results (RT-PCR) of the analysis of the expression of an inflammatory cytokine gene (MCP-1) in the case of adding various substances to bovine intestinal epithelial cells (after 12 hours). Their effects on LPS-stimulated increase in the expression of the inflammatory cytokine gene were examined. The ordinate indicates a relative expression level on the assumption that an expression level without addition of the various substances and without the LPS stimulation is set at 1.
[Figure 8] Figure 8 is a graph indicating the results (RT-PCR) of the analysis of the expression of an inflammatory cytokine gene (IL-8) in the case of adding various substances to bovine intestinal epithelial cells (after 12 hours). Their effects on LPS-stimulated increase in the expression of the inflammatory cytokine gene were examined. The ordinate indicates a relative expression level on the assumption that an expression level without addition of the various substances and without the LPS stimulation is set at 1.

### DETAILED DESCRIPTION OF THE INVENTION

The agent for suppressing inflammation in intestinal cells of the present invention comprises, as active components, cashew nut shell liquid (CNSL), heated cashew nut shell liquid, anacardic acid, cardanol, and/or cardol; and vitamin K.

Cashew nut shell liquid means non-heated cashew nut shell liquid when it is described as distinguished from heated cashew nut shell liquid.

Among cashew nut shell liquid (hon-heated), heated cashew nut shell liquid, anacardic acid, cardanol, and cardol, cashew nut shell liquid (hon-heated) and anacardic acid are preferable and cashew nut shell liquid (hon-heated) is more preferable.

Examples of suppression of inflammation in intestinal cells include suppression of an inflammatory symptom in intestinal cells or tissues, and suppression of production of an inflammatory cytokine in intestinal cells. The suppression of the production of the inflammatory cytokine includes not only suppression of secretion of an inflammatory cytokine but also suppression of expression of an inflammatory cytokine gene. Examples of the inflammatory cytokine include IL-6 (interleukine-6), MCP-1 (monocyte chemoattractant protein-1), IL-8 (interleukine-8), and TNF-α (tumor necrosis factor-α).

An intestinal epithelial cell is preferred as the intestinal cell.

The agent for suppressing of inflammation in intestinal cells of the present invention can also be used as an agent for suppressing production of an inflammatory cytokine in intestinal cells. The agent for suppressing production of an inflammatory cytokine preferably results in decreases in the production amount of the inflammatory cytokine to 90% or less, preferably 80% or less, and more preferably 50% or less, as compared with production amount in a control (without addition of CNSL, heated cashew nut shell liquid, anacardic acid, cardanol, and/or cardol; and vitamin K). The production amount may be the amount of the protein secreted from intestinal cells. However, the production amount is preferably gene expression level in the intestinal cells.

Suppression of production of an inflammatory cytokine, evoked by an inflammatory stimulus, is preferred as the suppression of production of an inflammatory cytokine. Examples of the inflammatory stimulus include a stimulus derived from bacteria, such as LPS (lipopolysaccharide). As a result, inflammation caused by a bacterial infection or the like can be efficiently suppressed.

The agent for suppressing of inflammation in intestinal cells and the agent for suppressing production of an inflammatory cytokine in intestinal cells, of the present invention, can be preferably used in prevention or treatment of a disease caused by inflammation in intestinal cells. Examples of such diseases include bacterial infections and inflammatory bowel diseases.

Cashew nut shell liquid is an oily liquid contained in the nutshell of the cashew nut tree (*Anacardium occidentale* L.). Cashew nut shell liquid contains, as its components, anacardic acid, cardanol, and cardol. Anacardic acid is converted into cardanol by heat treatment. Cashew nut shell liquid containing only cardanol and cardol due to heat treatment (heated cashew nut shell liquid) may also be used.

Cashew nut shell liquid (unheated) extracted by pressing cashew nut shells comprises 55 to 80% by mass of anacardic acid, 5 to 20% by mass of cardanol and 5 to 30% by mass of cardol, as described in J. Agric. Food Chem. 2001, 49, 2548-2551.

Heated cashew nut shell liquid obtained by heat treatment of unheated cashew nut shell liquid (for example, at 70°C or more, preferably 130°C or more), causing anacardic acid, which is a main component of unheated cashew nut shell liquid, to be decarboxylated and converted into cardanol, comprises 0 to 10% by mass of anacardic acid, 55 to 80% by mass of cardanol, and 5 to 30% by mass of cardol.

Cashew nut shell liquid can be obtained as a vegetable oil extracted by pressing cashew nut shells. Cashew nut shell liquid can also be obtained by dry distillation or solvent extraction of cashew nut shells. For example, cashew nut shell liquid can also be obtained by a method as described in Japanese Unexamined Patent Publication No. H08-231410. A commercial product of cashew nut shell liquid may be used.

The agent for suppressing inflammation in an intestinal cell and the agent for suppressing production of an inflammatory cytokine, of the present invention, may comprise one or more of anacardic acid, cardanol, and cardol, instead of cashew nut shell liquid.

Examples of anacardic acid include naturally-occurring anacardic acid, synthetic anacardic acid, and derivatives thereof. Moreover, a commercial product of anacardic acid may also be used. Anacardic acid can be obtained as described in Japanese Unexamined Patent Publication No. H08-231410 by extracting cashew nut oil from cashew nut shells treated with an organic solvent, subjecting the obtained cashew nut oil to, for example, silica gel column chromatography, and applying a mixed solvent of n-hexane, ethyl acetate and acetic acid with a varying ratio to the chromatography for the elution of the obtained cashew nut oil (see Japanese Unexamined Patent Publication No. H03-240721, Japanese Unexamined Patent Publication No. H03-240716, etc.).

Examples of cardanol include naturally-occurring cardanol, synthetic cardanol, and derivatives thereof. Moreover, cardanol to be used in the present invention can be obtained by decarboxylation of anacardic acid, a main component of cashew nut shell liquid.

Examples of cardol include naturally-occurring cardol, synthetic cardol, and derivatives thereof. Moreover, cardol to be used in the present invention can be obtained from purification of cashew nut shell liquid.

The content (sum amount in the case of comprising two or more) of cashew nut shell liquid, heated cashew nut shell liquid, anacardic acid, cardanol, and/or cardol in the agent for suppressing inflammation in intestinal cells or the agent for suppressing production of an inflammatory cytokine, of the present invention, is, for example, 1% by mass to 95% by mass based on the total amount of the agent.

The kind of vitamin K is not particularly limited, and examples thereof include vitamin K1, vitamin K2, vitamin K3, vitamin K4, and vitamin K5. However, the vitamin K is more preferably vitamin K1.

The content of vitamin K in the agent for suppressing inflammation in intestinal cells or the agent for suppressing production of an inflammatory cytokine, of vitamin K, is, for example, 0.0001% by mass to 20% by mass, 0.001% by mass to 20% by mass, or 0.01% by mass to 20% by mass based on the total amount of the agent.

The agent for suppressing inflammation in intestinal cells and the agent for suppressing production of an inflammatory cytokine, of the present invention, may comprise components such as an excipient which can be used in a feed or pharmaceutical product, such as, for example, lactose, sucrose, D-mannitol, α-starch, starch, corn starch, crystalline cellulose, bentonite, silica gel, or light anhydrous silicic acid, in addition to cashew nut shell liquid, heated cashew nut shell liquid, anacardic acid, cardanol, and/or cardol, and vitamin K.

The agent for suppressing inflammation in intestinal cells and the agent for suppressing production of an inflammatory cytokine, of the present invention, may further comprise an optional component such as a component effective in promotion of the growth of a ruminant, a nutritional component, a component enhancing storage stability, or a coating component, in addition to cashew nut shell liquid, heated cashew nut shell liquid, anacardic acid, cardanol, and/or cardol, and vitamin K. Examples of such optional components include raw materials of feeds, such as bran, alfalfa, and timothy, feed additives, food source materials, food additives, pharmaceutical product materials, and other supplement components used in supplements for animals (hereinafter referred to as "supplements"). Examples thereof include: living bacterial agents such as bacteria belonging to the genus *Enterococcus*, bacteria belonging to the genus *Bacillus*, and *Lactobacillus bifidus*; enzymes such as amylase and lipase; L-ascorbic acid, choline chloride, inositol, and folic acid; minerals such as potassium chloride, ferric citrate, magnesium oxide, and phosphates; amino acids such as DL-alanine, DL-methionine, and L-lysine; organic acids such as fumaric acid, butyric acid, lactic acid, and acetic acid, and salts thereof; antioxidants such as ethoxyquin, dibutyl hydroxytoluene, butylated hydroxyanisole, ferulic acid, vitamin C, and vitamin E; fungicides such as calcium propionate; binding agents such as carboxymethyl cellulose (CMC), sodium caseinate, and sodium polyacrylate; emulsifiers such as lecithin, glycerin fatty acid ester, and sorbitan fatty acid ester; coloring agents such as astaxanthin and canthaxanthin; and flavoring agents such as various esters, ethers, and ketones.

The agent for suppressing inflammation in intestinal cells and the agent for suppressing production of an inflammatory cytokine, of the present invention, may comprise an oil absorption agent such as magnesium oxide, stearate, talc, zeolite, diatom earth, or silica, and the oil absorption agent is preferably granulous. The oil absorption agent is preferably an oil absorption agent that can adsorb 50 to 300 g of oil per 100 g of the oil absorption agent. When the oil absorption agent has a particle diameter of more than 300 µm, the particles of the oil absorption agent are coarsened and separated. Therefore, the oil absorption agent preferably has a particle diameter of 2 to 300 µm.

In one embodiment of the agent for suppressing inflammation in intestinal cells and the agent for suppressing production of an inflammatory cytokine, of the present invention, a preferred mass ratio between the oil absorption agent and cashew nut shell liquid, heated cashew nut shell liquid, anacardic acid, cardanol, and/or cardol, and vitamin K is 100:20 to 100:180.

The dosage form for the agent for suppressing inflammation in intestinal cells and the agent for suppressing production of an inflammatory cytokine, of the present invention, is not particularly limited, but examples thereof include any forms such as liquid medicine, powder, solid, tablet, capsule, emulsion, pellet, tablet, and coating. The dosage form is preferably liquid medicine, powder, capsule, pellet, or tablet.

Cashew nut shell liquid, heated cashew nut shell liquid, anacardic acid, cardanol, and/or cardol may be used on an as-is basis as the liquid medicine and combined with vitamin K. Alternatively, cashew nut shell liquid, heated cashew nut shell liquid, anacardic acid, cardanol, and/or cardol is/are dissolved in a solvent such as ethanol, and the solution may be combined with vitamin K. Further, the above-described excipient or an optional component may be added thereto. The powder, capsule, pellet, or tablet as described below may be suspended and allowed to float in a liquid.

As for the powder, the above-described excipient may be added to cashew nut shell liquid, heated cashew nut shell liquid, anacardic acid, cardanol, and/or cardol, and vitamin K to make the powder.

As for the capsule, a capsule may be filled with cashew nut shell liquid, heated cashew nut shell liquid, anacardic acid, cardanol, and/or cardol, and vitamin K on an as-is basis, or the above-described excipient or an optional component may be added to the capsule.

As for the pellet, the above-described excipient may be added to cashew nut shell liquid, heated cashew nut shell liquid, anacardic acid, cardanol, and/or cardol, and vitamin K and the resultant may be granulated to make the pellet.

As for the tablet, the above-described excipient may be added to cashew nut shell liquid, heated cashew nut shell liquid, anacardic acid, cardanol, and/or cardol, and vitamin K and the resultant may be granulated to make the tablet.

It is preferable to formulate the agent as the powder, the tablet, and the pellet when the agent comprises an oil absorption agent such as silica.

As described above, the agent for suppressing inflammation in intestinal cells and the agent for suppressing production of an inflammatory cytokine, of the present invention, can be produced by mixing cashew nut shell liquid, heated cashew nut shell liquid, anacardic acid, cardanol, and/or cardol with vitamin K, and further with an excipient or an optional component, as necessary, for formulation. In addition, according to the form of the agent, a product obtained by pulverizing/crushing the cashew nut shell, or cashew nut shell without being processed may be mixed directly with vitamin K and any other optional components to produce the agent for suppressing inflammation in intestinal cells and the agent for suppressing production of an inflammatory cytokine, of the present invention.

It is preferable to mix cashew nut shell liquid, heated cashew nut shell liquid, anacardic acid, cardanol, and/or cardol, and vitamin K to a feed for an animal to be administered.

Accordingly, the present invention provides a feed for suppressing intestinal inflammation of a non-human animal, comprising, as active components, cashew nut shell liquid, heated cashew nut shell liquid, anacardic acid, cardanol, and/or cardol, and vitamin K.

The feed of the present invention comprises cashew nut shell liquid, heated cashew nut shell liquid, anacardic acid, cardanol, and/or cardol, and vitamin K.

The content (sum amount in the case of comprising two or more) of cashew nut shell liquid, heated cashew nut shell liquid, anacardic acid, cardanol, and/or the cardol in the feed of the present invention is preferably 1 to 1000 ppm by weight, more preferably 1 to 500 ppm by weight, still more preferably 1 to 300 ppm by weight, and even more preferably 5 to 300 ppm, and particularly preferably 5 to 100 ppm by weight based on the total amount per dry matter mass of the feed.

The content of vitamin K in the feed of the present invention is preferably 1 to 20 ppm by weight, more preferably 1 to 10 ppm by weight, and still more preferably 1 to 5 ppm by weight based on the total amount per dry matter mass of the feed.

It is preferable that, in the feed of the present invention, the content (sum amount in the case of comprising two or more) of cashew nut shell liquid, heated cashew nut shell liquid, anacardic acid, cardanol, and/or the cardol is 5 to 100 ppm by weight based on the total amount per dry matter mass of the feed and the content of vitamin K is 1 to 10 ppm by weight based on the total amount per dry matter mass of the feed.

It is preferable that, in the feed of the present invention, the weight ratio of the content (sum amount in the case of comprising two or more) of cashew nut shell liquid, heated cashew nut shell liquid, anacardic acid, cardanol, and/or the cardol and the content of vitamin K is in the range of 30:1 to 30:10.

The feed of the present invention can be produced by adding cashew nut shell liquid, heated cashew nut shell liquid, anacardic acid, cardanol, and/or cardol, and vitamin K, or the agent for suppressing inflammation in an intestinal cell of the present invention, comprising cashew nut shell liquid, heated cashew nut shell liquid, anacardic acid, cardanol, and/or cardol, and vitamin K to a feed component, and by mixing the resultant.

In such a case, when the agent which is powdery and solid is used, the agent may be allowed to be in a liquid or a gel form using a liquid carrier to facilitate the mixture. In this case, a flowable liquid such as water, vegetable oil, liquid animal oil, mineral oil, synthetic oil, or a water-soluble polymer compound can be used as the liquid carrier. Moreover, water-soluble polysaccharides such as alginic acid, sodium alginate, xanthan gum, carboxymethyl cellulose, α-starch, sodium caseinate, gum arabic, guar gum, and tamarind seed polysaccharides are also preferably combined to keep the homogeneity of cashew nut shell liquid, heated cashew nut shell liquid, anacardic acid, cardanol, and/or cardol, and vitamin K in the feed.

For the feed of the present invention, the kinds, combination rates, and the like of feed components combined with the agent of the present invention are not particularly limited. The feed may be a feed conventionally given to each animal. The feed can be prepared using, for example, corn grains, corn flours, milo, bran, soybean cake, oats, wheat short, wheat coarse powders, alfalfa, timothy, clovers, defatted rice bran, northern ocean meal, coast meal, yeast, molasses, meat pieces, bone meal, calcium carbonate, calcium diphosphate, yellow grease, vitamins, minerals, and/or the like.

Moreover, the present invention provides a method for suppressing inflammation in the intestinal cells of a non-human animal, the method comprising administering cashew nut shell liquid, heated cashew nut shell liquid, anacardic acid, cardanol, and/or cardol, and vitamin K to the non-human animal.

It is preferable to mix cashew nut shell liquid, heated cashew nut shell liquid, anacardic acid, cardanol, and/or cardol, and vitamin K to a feed, and to administer the feed to the non-human animal. The preferred ranges of the amounts of cashew nut shell liquid, heated cashew nut shell liquid, anacardic acid, cardanol, and/or cardol, and vitamin K combined with the feed are as described above.

The kinds of non-human animals to administer the feed of the present invention are not particularly limited, but are preferably non-human mammalian animals and poultry, and examples thereof include ruminants, pigs, and poultry. Examples of the ruminants include cattle, buffalos, goats, sheep, and yaks. Examples of the kinds of the cattle include, but are not limited to, female Holstein, Jersey, Japanese black, Japanese Shorthorn, and Aberdeen Angus. Examples of the poultry include chickens, quails, turkeys, ducks, gooses, ostriches, emus, pheasants, and guinea fowl.

The administration of cashew nut shell liquid, heated cashew nut shell liquid, anacardic acid, cardanol, and/or cardol, and vitamin K results in suppression of production of an inflammatory cytokine in intestinal cells to suppress inflammation in the intestinal cell of the non-human animal.

The amount of the feed to be given may be appropriately adjusted depending on the kind, body weight, age, sex, and health condition of the animal, feed components, and the like. In this case, the amounts (sum amount in the case of comprising two or more) of cashew nut shell liquid, heated cashew nut shell liquid, anacardic acid, cardanol, and/or cardol contained in the feed are, for example, 0.1 to 50 g/animal/day, and preferably 0.5 to 5 g/animal/day, and the amount of vitamin K is, for example, 0.01 to 5 g/animal/day, and preferably 0.05 to 0.5 g/animal/day.

### EXAMPLES

The present invention will be specifically described below with reference to Examples. However, aspects of the present invention are not limited to the following Examples.

To evaluate the effect of endotoxin stimulated inflammatory reaction in the intestine, CNSL, vitamin K1 (FUJIFILM Wako Pure Chemical Corporation), or a combination thereof was added to bovine intestinal epithelial cells, the cells were cultured, and cytokine gene expression analysis was performed. Aspirin was used as a positive control.

### Testing method

Evaluation of Cell Culture, LPS Stimulation, and Anti-inflammatory Substance
1. Seeding of BIE Cells
   BIE (bovine intestinal epithelial) cells were seeded in a 12-well plate (manufactured by COSTAR) at 3.0 × 10⁴ cells/well, and cultured in a CO₂ incubator. (Day 0)
2. Added Samples
   · Medium (DMEM Dulbecco's modified MEM medium) as NC (negative control)
   ·Acetylsalicylic acid (aspirin)
      Aspirin (manufactured by BAYER) was ground in a mortar, and PBS (phosphate buffered saline) solution containing 10 mg/mL of aspirin was then produced and sterilized through a filter of 0.45 µm (manufactured by ADVANTEC TOYO). The resultant was diluted with the sterilized PBS to 500 µg/mL, and added in 1/50 volume to the medium. (Final concentration of 10 ppm)
   ·Vitamin K1
      Ethanol solution containing 50 mg/mL of vitamin K1 was prepared, and the solution was diluted with PBS to 0.25 mg/mL, and then sterilized through a filter of 0.45 µm (manufactured by ADVANTEC TOYO). The resultant was added in 1/50 volume to the medium. (Final concentration of 5 ppm)
   ·CNSL (cashew nut shell liquid)
      → Ethanol solution (stock solution) containing 50 mg/mL (CNSL) was prepared, and the solution was diluted with PBS to produce 1.5 mg/mL of solution, which was then sterilized through a filter (0.45 µm). It was diluted with the medium to 1/50 volume (Final concentration of 30 ppm).
3. Medium Replacement
   20 µL of the above-described samples was added to 1 mL of DMEM to prepare a medium for replacement.
   The medium in the 12-well plate was aspirated and replaced with the medium for replacement (at 9:00 a.m. on Day 3)

### LPS Stimulation

An LPS (lipopolysaccharide) solution was added to the medium to achieve a final concentration of 1 µg/mL (at 9:00 a.m. on Day 5).

### Harvest

A culture supernatant was collected, the wells were then washed with the sterilized PBS, and adherent cells were solubilized with TRIZOL, and harvested (at 9:00 p.m. on Day 5 and at 9:00 a.m. on Day 6) (12 hours or 24 hours)

### RT-PCR

The sample in which TRIZOL (manufactured by INVITROGEN) is dissolved is partially purified by phenol-chloroform extraction and ethanol precipitation, and then dissolved in 10 µL of RNase-free water, and the O.D. (absorbance) of the resultant is measured with NanoDrop (manufactured by Thermo Fisher Scientific K.K.).

Real time PCR (IL-6, IL-8, MCP-1, and TNF-α) was performed in conformity with the manual of Prime Script RT reagent Kit with g DNA Eraser of TAKARABIO.

### Results

The results of RT-PCR are set forth in Figures 1 to 7. The results reveal that LPS-induced increases in the expression of IL-6, IL-8, MCP-1, and TNF-α were efficiently suppressed by combining CNSL with vitamin K.

The similar experiment (CNSL 30ppm) was conducted by changing the concentration of vitamin K1 (2.5ppm or 10ppm) to analyze the expression amount of IL-8 in the bovine intestinal epithelial cells. The result of Fig. 8 shows that LPS-induced increase in the expression of IL-8 was suppressed by combining CNSL with vitamin K at 2.5ppm and 10ppm.

### Discussion

It was suggested that CNSL and aspirin (acetylsalicylic acid) suppress the action of the inflammation mediators, stimulated by endotoxin (LPS), in BIE cells (bovine intestinal epithelial cells). Moreover, it was found that the effect is synergistically enhanced by combining with vitamin K.

## Claims

1. A method of suppressing inflammation in intestinal cells of a non-human animal, comprising administering cashew nut shell liquid, heated cashew nut shell liquid, anacardic acid, cardanol, and/or cardol, and vitamin K to a non-human animal.

2. The method according to claim 1, comprising: mixing a feed with cashew nut shell liquid, heated cashew nut shell liquid, anacardic acid, cardanol, and/or cardol, and vitamin K; and administering the feed to a non-human animal,

3. The method according to claim 2, comprising: mixing the feed with 1 ppm to 1000 ppm (weight) of cashew nut shell liquid, heated cashew nut shell liquid, anacardic acid, cardanol, and/or cardol, and 1 to 20 ppm (weight) of vitamin K; and administering the feed to the non-human animal.

4. The method of suppressing inflammation in intestinal cells according to any one of claims 1 to 3, wherein the non-human animal is a mammal.

5. The method of suppressing inflammation in intestinal cells according to any one of claims 1 to 3, wherein the non-human animal is poultry, a pig, or a ruminant.

6. The method of suppressing inflammation in intestinal cells according to any one of claims 1 to 3, wherein the non-human animal is cattle.

7. The method of suppressing inflammation in intestinal cells in a non-human animal according to any one of claims 1 to 6, wherein the administration of cashew nut shell liquid, heated cashew nut shell liquid, anacardic acid, cardanol, and/or cardol, and vitamin K results in suppression of production of an inflammatory cytokine in intestinal cells to suppress inflammation in intestinal cells of the non-human animal.

8. The method according to claim 7, wherein the inflammatory cytokine is one or more of TNFα, IL-6, IL-8, and MCP-1.

9. The method according to any one of claims 1 to 8, wherein the vitamin K is vitamin K1.

10. The method according to any one of claims 1 to 9, said method comprising administering cashew nut shell liquid and/or anacardic acid, and vitamin K to a nonhuman animal.

11. An agent for suppressing intestinal inflammation of a non-human animal, comprising, as active components, cashew nut shell liquid, heated cashew nut shell liquid, anacardic acid, cardanol, and/or cardol, and vitamin K.

12. The agent for suppressing intestinal inflammation according to claim 11, wherein the vitamin K is vitamin K1.

13. The agent for suppressing intestinal inflammation according to claim 11 or 12, wherein said agent comprises, as active components, cashew nut shell liquid and/or anacardic acid, and vitamin K.

14. An agent for suppressing production of an inflammatory cytokine in intestinal cells of a non-human animal, comprising, as active components, cashew nut shell liquid, heated cashew nut shell liquid, anacardic acid, cardanol, and/or cardol, and vitamin K.

15. The agent for suppressing production of an inflammatory cytokine according to claim 14, wherein the vitamin K is vitamin K1.

16. The agent for suppressing production of an inflammatory cytokine according to claim 14 or 15, wherein said agent comprises, as active components, cashew nut shell liquid and/or anacardic acid, and vitamin K.

17. A feed for suppressing intestinal inflammation of a non-human animal, comprising, as active components, cashew nut shell liquid, heated cashew nut shell liquid, anacardic acid, cardanol, and/or cardol, and vitamin K.

18. The feed according to claim 17, comprising 1 ppm to 1000 ppm (weight) of cashew nut shell liquid, heated cashew nut shell liquid, anacardic acid, cardanol, and/or cardol, and 1 to 20 ppm (weight) of vitamin K.

19. The feed according to claim 17, comprising 5 ppm to 100 ppm (weight) of cashew nut shell liquid, heated cashew nut shell liquid, anacardic acid, cardanol, and/or cardol, and 1 to 10 ppm (weight) of vitamin K.

20. The feed according to claim 17, wherein the weight ratio of the content of cashew nut shell liquid, heated cashew nut shell liquid, anacardic acid, cardanol, and/or the cardol and the content of vitamin K is in the range of 30:1 to 30: 10.

21. The feed according to any one of claims 17 to 20, said feed comprising cashew nut shell liquid and/or anacardic acid, and vitamin K.
